# EUROPEAN PATENT SPECIFICATION

(11) **EP 3 345 532 B1**
(45) Date of publication and mention of the grant of the patent: **20.04.2022**
(21) Application number: 17195416.7
(22) Date of filing: 09.10.2017
(51) Int. Cl.: A61B 3/13, A61B 3/10, A61B 3/14, A61B 90/20, G02B 21/00

(54) **OBSERVATION SYSTEM AND OBSERVATION CONTROL PROGRAM**
BEOBACHTUNGSSYSTEM UND BEOBACHTUNGSSTEUERUNGSPROGRAMM
SYSTÈME D'OBSERVATION ET PROGRAMME DE COMMANDE D'OBSERVATION

(30) Priority: 13.10.2016 JP 2016201881
(43) Date of publication of application: 11.07.2018
(73) Proprietor: Nidek Co., Ltd., Gamagori-shi, Aichi, 443-0038 (JP)
(72) Inventor: MATSUNOBU, Go, Gamagori-shi, Aichi 443-0038 (JP)
(74) Representative: Viering, Jentschura & Partner mbB Patent- und Rechtsanwälte

(56) References cited:
- WO-A2-2009/033110
- JP-A- 2015 163 092
- US-A1- 2015 223 685
- US-A1- 2016 008 169

## Description

### TECHNICAL FIELD

The present disclosure relates to an observation system and an observation control program that are used for observing a biological object during surgery.

### BACKGROUND

Various systems that allow a user (such as a surgeon) to observe a biological object during surgery are known. For example, Japanese Laid-Open Patent Publication No. 2015-163092 discloses a technique in which an OCT device using optical coherence tomography (OCT) and a surgical microscope, which performs magnified display of an interior of a patient's eye, are used simultaneously during surgery. WO 2009/033110 A2 discloses an observation system comprising a surgical microscope and and OCT unit; the microscope comprises a focussing objective lens, a beam splitter and means for controlling and moving the objective lens, the objective lens being also adapted to adjust the focus of the OCT measurement beam.

### SUMMARY

For example, there are cases in which, during the surgery, a surgeon changes a position that the surgeon wishes to observe, or the biological object moves. In this type of case, with the surgical microscope, it is necessary to adjust the focus of an observation optical system. Further, with the OCT device, it is necessary to perform an adjustment of the OCT light (such as adjustment of an optical path length difference between a measurement luminous flux and a reference luminous flux). In the known observation system that includes the OCT device and the surgical microscope, the adjustment of the OCT light of the OCT device is performed independently from the adjustment of the focus of the surgical microscope. Thus, with the observation system, it has been difficult to perform the adjustment of the OCT light in a rapid and easy manner.

A typical object of the present disclosure is to provide an observation system and an observation control program that can rapidly and easily perform adjustment of OCT light.

The present invention provides an observation system according to claim 1, and an observation control program according to claim 12. Further embodiments of the present invention are disclosed in the dependent claims.

An observation system according to a first aspect of typical embodiments of the present disclosure includes a surgical microscope, an OCT unit adapted to acquire an OCT signal, and a processor, the surgical microscope includes an observation optical system adapted to optically guide an observation luminous flux from a biological object being an observation object, and an observation focus adjustment unit provided on an optical path of the observation luminous flux in the observation optical system, the observation focus adjustment unit being adapted to adjust a focus of the observation optical system, the OCT unit includes an OCT light source, a light splitter adapted to split a luminous flux emitted from the OCT light source into a measurement luminous flux and a reference luminous flux, a photodetection element adapted to receive an interference light obtained by synthesis of the reference luminous flux and a measurement luminous flux reflected by the biological object, and an optical path length difference adjustment unit provided on at least one of an optical path of the measurement luminous flux and an optical path of the reference luminous flux, the optical path length difference adjustment unit being adapted to adjust an optical path length difference between the measurement luminous flux and the reference luminous flux, and the processor is adapted to drive the optical path length difference adjustment unit of the OCT unit in accordance with a change in a focus state of the observation optical system.

An observation control program according to a second aspect of typical embodiments of the present disclosure is executed by a control unit adapted to control an observation system that includes a surgical microscope and an OCT unit adapted to acquire an OCT signal, the surgical microscope includes an observation optical system adapted to optically guide an observation luminous flux from a biological object being an observation object, and an observation focus adjustment unit provided on an optical path of the observation luminous flux in the observation optical system, the observation focus adjustment unit being adapted to adjust a focus of the observation optical system, the OCT unit includes an OCT light source, a light splitter adapted to split a luminous flux emitted from the OCT light source into a measurement luminous flux and a reference luminous flux, a photodetection element adapted to receive an interference light obtained by synthesis of the reference luminous flux and a measurement luminous flux reflected by the biological object, and an optical path length difference adjustment unit provided on at least one of an optical path of the measurement luminous flux and an optical path of the reference luminous flux, the optical path length difference adjustment unit being adapted to adjust an optical path length difference between the measurement luminous flux and the reference luminous flux, and the observation control program, when executed by a processor of the control unit, causes the control unit to perform an optical path length difference adjustment step of driving the optical path length difference adjustment unit of the OCT unit in accordance with a change in a focus state of the observation optical system.

According to the observation system and the observation control program according to the present disclosure, the adjustment of OCT light can be rapidly and easily performed.

An observation system exemplified in the present disclosure includes a surgical microscope, an OCT unit, and a processor. The surgical microscope includes an observation optical system and an observation focus adjustment unit. The observation optical system optically guides an observation luminous flux from a biological object that is an observation object. The observation focus adjustment unit is provided on an optical path of the observation luminous flux in the observation optical system, and adjusts a focus of the observation optical system (hereinafter referred to as an "observation focus"). The OCT unit includes an OCT light source, a light splitter, a photodetection element, and an optical path length difference adjustment unit. The OCT light source emits a OCT light. The light splitter splits a luminous flux emitted from the OCT light source into a measurement luminous flux and a reference luminous flux. The photodetection element receives an interference light obtained by synthesis of the reference luminous flux and a measurement luminous flux reflected by the biological object. The optical path length difference adjustment unit is provided on at least one of an optical path of the measurement luminous flux and an optical path of the reference luminous flux, and adjusts an optical path length difference between the measurement luminous flux and the reference luminous flux. The processor drives the optical path length difference adjustment unit of the OCT unit in accordance with a change in a focus state of the observation optical system.

In this case, even if the biological object moves, an observation position changes, or the like, the optical path length difference of the OCT unit can be appropriately adjusted in accordance with the change in the focus state of the observation optical system of the surgical microscope (a microscope observation optical system). Thus, the adjustment of the OCT light can be rapidly and appropriately performed.

When the adjustment of the optical path length difference of the OCT unit is not linked to the change in the focus state of the observation optical system, as in a conventional way, it is conceivable that the processor of the OCT unit only performs automatic optical path length (OPL) adjustment that searches for a position at which a tomographic image is acquired while changing the optical path length difference. However, in this case, it is necessary for the processor to search over a wide range while causing the optical path length difference to significantly vary. In contrast to this, the observation system according to the present disclosure, for example, can link the adjustment of the optical path length difference of the OCT unit to the adjustment of the observation focus, which can be adjusted in a shorter time than the automatic optical path length adjustment. As a result, the adjustment of the OCT light can be performed rapidly and appropriately.

A specific method for driving the optical path length difference adjustment unit in accordance with the change in the focus state of the observation optical system may be selected as appropriate. For example, the processor may calculate a movement amount ΔZ by which a position of an object to be observed has moved, on the basis of a drive amount of the observation focus adjustment unit, and may determine a drive amount of the optical path length difference adjustment unit on the basis of the calculated ΔZ. The processor may use a ratio between a movement amount of the observation focus corresponding to the drive amount of the observation focus adjustment unit and a change amount of the optical path length difference corresponding to the drive amount of the optical path length difference adjustment unit. In this case, the processor may determine the drive amount of the optical path length difference adjustment unit depending on the drive amount of the observation focus adjustment unit and the above-described ratio. The processor may detect a deviation of a current observation focus in relation to an appropriate observation focus, and may determine the drive amount of the optical path length difference adjustment unit on the basis of the deviation of the detected observation focus. In this case also, the optical path length difference can be appropriately adjusted in accordance with the change in the focus state of the observation optical system. The processor may drive the optical path length difference adjustment unit after driving the observation focus adjustment unit. When the deviation amount of the observation focus can be acquired before driving the observation focus adjustment unit (when the observation focus state can be acquired by a phase-difference detection method to be described below, for example) or the like, the observation focus adjustment unit and the optical path length difference adjustment unit may be simultaneously linked and driven.

The observation system may be a single device in which the surgical microscope and the OCT unit are integrated. The observation system may be a system that includes the surgical microscope and an OCT device that is a device separate from the surgical microscope. The processor that controls the adjustment of the OCT light may be a processor provided in the OCT unit or may be a processor provided in the surgical microscope. A processor of a personal computer or the like connected to the surgical microscope and the OCT unit may control the adjustment of the OCT light. Processors provided in a plurality of devices (such as the surgical microscope and the OCT device) may work in concert to control the adjustment of the OCT light. In this case, the plurality of devices function as a control device that controls the observation system.

The processor may acquire the focus state of the observation optical system of the surgical microscope. The processor may drive the observation focus adjustment unit and the optical path length difference adjustment unit on the basis of an acquisition result of the focus state of the observation optical system. In other words, the processor may link the driving of the observation focus adjustment unit and the driving of the optical path length difference adjustment unit while performing automatic focusing of the observation optical system. In this case, the optical path length difference of the OCT light can also be appropriately adjusted while the automatic focusing of the observation optical system is performed. Thus, a microscopic image and an OCT signal can be appropriately acquired.

A specific method for driving the optical path length difference adjustment unit on the basis of an acquisition result of the focus state of the observation optical system may be selected as appropriate. For example, the processor may calculate the drive amount of the optical path length difference adjustment unit on the basis of the acquisition result of the focus state of the observation optical system. The processor may calculate the drive amount of the observation focus adjustment unit on the basis of the acquisition result of the focus state of the observation optical system, and may calculate the drive amount of the optical path length difference adjustment unit on the basis of the drive amount of the observation focus adjustment unit.

The automatic focusing of the observation optical system may be constantly performed during the observation of the biological object using the surgical microscope. The processor may acquire the focus state of the observation optical system and perform the automatic focusing when a command has been input by a user that causes the automatic focusing of the observation optical system to be performed. In this case, the user can appropriately observe the biological object by appropriately inputting the command to perform the automatic focusing at a timing at which the user wishes to bring the observation optical system into focus.

In addition, the focus of the observation optical system may be adjusted in accordance with an operation of the user. In this case also, the processor can rapidly and appropriately perform the adjustment of the OCT light, by driving the optical path length difference adjustment unit in accordance with the change in the focus state of the observation optical system.

The surgical microscope may include an imaging device that captures a microscopic image of the biological object by receiving the observation luminous flux optically guided by the observation optical system. The processor may cause a display to display the microscopic image captured by the imaging device, and may acquire the focus state of the observation optical system on the basis of a signal from the imaging device. In this case, the imaging device for capturing the microscopic image can be used to acquire the focus state of the observation optical system. Thus, the observation system can appropriately acquire the focus state while suppressing an increase in a dedicated configuration for acquiring the focus state of the observation optical system.

As a method for acquiring the focus state of the observation optical system on the basis of the signal from the imaging signal, various methods may be applied.

For example, the processor may acquire the focus state by a contrast detection method. In this case, the processor may acquire the focus state by analyzing the microscopic image captured by the imaging device while changing the observation focus and causing a position at which the contrast of the microscopic image is high to be the position at which the observation focus is in focus.

The processor may acquire the focus state using image surface phase-difference detection method. In this case, a phase difference pixel, which is formed in a non-symmetrical shape in the left-right direction, is embedded among pixels of the imaging device in order to detect a phase difference (parallax) of the image. The processor calculates the phase difference on the basis of a signal obtained by light injected from one of the left and right directions being selectively received by the phase difference pixel. The processor may acquire the focus state by causing a position at which the phase difference is smaller to be the position at which the observation focus is in focus.

As a method for acquiring the focus state, a method that does not use the signal from the imaging device may be applied. When the method is applied that does not use the signal from the imaging device, the surgical microscope may be a surgical microscope that allows the user to observe the biological object via an ocular lens, without being provided with the imaging device.

For example, a phase-difference detection method may be applied that uses a phase-difference detection sensor separate from the imaging device of the observation system that captures the microscopic image. In this case, the surgical microscope may include a separator lens that creates two images from the observation luminous flux, and a phase-difference detection sensor for detecting the phase difference (the parallax) from the two images. The processor may calculate the phase difference on the basis of a signal obtained using the phase-difference detection sensor, and may acquire the focus state by causing the position at which the phase difference is smaller to be the position at which the observation focus is in focus.

Further, for example, the focus state may be acquired using an astigmatism detection method, a knife edge method, the Foucault method, a critical angle method or the like. The astigmatism detection method is a method that detects the focus state using an astigmatism that occurs due to a difference between focal positions of a cylindrical lens and an objective lens. The knife edge method is a method in which a wall (knife edge) that blocks light on one surface of an optical path is provided on an objective lens focal point between the objective lens and a two-way photodiode and the focus state is detected by an amount of light injected into the two-way photodiode. The Foucault method is a method that detects the focus state by detecting a change in the optical path occurring due to a relationship between a focal position of an objective lens and a position of a prism surface, using two two-way photodiodes. The critical angle method is a method that detects the focus state by detecting, using a two-way photodiode, a change in a ratio of reflected light and transmitted light, using a critical angle prism.

The processor may set an interest region (including a point and a line) within a region of the captured microscopic image. The processor may acquire the focus state of the observation optical system on the basis of a signal for an evaluation region, which is a region, of the microscopic image, that includes the set interest region. In this case, by performing the automatic focusing of the observation optical system, the image quality of the interest region can be favorably maintained. The user can therefore more appropriately observe the biological object.

A specific method for setting the interest region may be selected as appropriate. For example, the processor may set the interest region at a position specified on the basis of a command from the user input to specify the interest region. The processor may automatically set the interest region by performing image processing on the captured microscopic image and detecting a particular portion of the biological object included in the microscopic image (in the case of the microscopic image of the ocular fundus, the macula lutea, the optic disc, or a fundus oculi blood vessel, for example). A particular region within a captured image region (such as a central region, for example) may be set in advance as the interest region. The interest region and the evaluation region may match each other.

The processor can also detect the focus state of the observation optical system on the basis of a signal for the whole image region of the captured microscopic image. In this case, the image quality of the whole of the microscopic image can be uniformly improved.

The processor may set a position at which the OCT measurement luminous flux is caused to scan (hereinafter referred to as a "scanning position"), on the basis of at least one of the interest region and the evaluation region. In this case, the scanning position of the measurement luminous flux is linked to at least one of the interest region and the evaluation region. Thus, the user can observe a desire position using the high quality microscopic image and the high quality OCT image.

The scanning position of the OCT measurement luminous flux may be set independently from the interest region and the evaluation region. For example, a configuration may be adopted such that the user can separately specify the interest region in the microscopic image and the scanning position of the OCT measurement luminous flux. The processor may set at least one of the interest region and the evaluation region on the basis of the specified scanning position after the scanning position of the OCT measurement luminous flux has been specified by the user. For example, the processor may set a region that includes the specified scanning position as the evaluation region.

By performing image processing on the captured microscopic image, the processor may detect a movement of a subject captured in the microscopic image (such as the biological object or a position of an aperture of a front lens arranged in a fixed position with respect to the biological object). The processor may cause the position of the evaluation region in the microscopic image to be tracked (perform tracking) in accordance with the movement of the detected subject. In this case, even if the subject moves, the image quality of the interest region can be favorably maintained.

The processor may drive the optical path length difference adjustment unit on the basis of an analysis result of the OCT signal and a change in the focus state of the observation optical system. In this case, the optical path length difference adjustment unit is driven while taking into account the analysis result of the actually acquired OCT signal. Thus, the adjustment of the optical path length difference can be more appropriately performed.

In this case, a specific method for controlling the optical path length difference adjustment unit may be selected as appropriate. For example, the processor may drive the optical path length difference adjustment unit on the basis of the analysis result of the OCT signal (at a position at which the level of the OCT signal is equal to or greater than a threshold value, for example), after driving the optical path length difference adjustment unit to a first position on the basis of a drive amount of the observation focus adjustment unit. The processor may determine a position at which the driving is stopped by referring to the analysis result of the OCT signal while driving the optical path length difference adjustment unit toward the first position on the basis of the drive amount of the observation focus adjustment unit. The optical path length difference adjustment unit may be driven without using the analysis result of the OCT signal.

When the biological object moves, an observation position changes, or the like, there may be a case in which it is preferable to also adjust the focus (hereinafter referred to as an "OCT focus") of the OCT measurement luminous flux. The processor is adapted to drive an OCT focus adjustment unit in accordance with the change in the focus state of the observation optical system. In this case, the OCT focus can be rapidly and appropriately adjusted along with the optical path length difference.

The processor may drive the OCT focus adjustment unit on the basis of an analysis result of the OCT signal and a change in the focus state of the observation optical system. In this case, the OCT focus adjustment unit is driven while taking into account the analysis result of the actually acquired OCT signal. Thus, the adjustment of the OCT focus can be more appropriately performed.

In this case, a specific method for controlling the OCT focus adjustment unit may be selected as appropriate. For example, the processor may drive the OCT focus adjustment unit on the basis of the analysis result of the OCT signal (at a position at which the level of the OCT signal is equal to or greater than a threshold value, for example), after driving the OCT focus adjustment unit to a first position on the basis of a drive amount of the observation focus adjustment unit. The processor may determine a position at which the driving is stopped by referring to the analysis result of the OCT signal while driving the OCT focus adjustment unit toward the first position on the basis of the drive amount of the observation focus adjustment unit. The OCT focus adjustment unit may be driven without using the analysis result of the OCT signal.

The processor may acquire an optical parameter of the observation optical system, and may drive at least one of the optical path length difference adjustment unit and the OCT focus adjustment unit on the basis of the acquired optical parameter. In this case, even if the optical parameter (a variable focal length, for example) of the observation optical system is changed, the OCT light can be appropriately adjusted in accordance with the changed optical parameter.

The processor may change a drive amount of at least one of the optical path length difference adjustment unit and the OCT focus adjustment unit, depending on whether a wide angle observation unit, which widens an observation angle of view of the ocular fundus, is being used. In this case, the OCT light that is linked to the change in the focus state of the observation optical system can be appropriately adjusted depending on whether the wide angle observation unit is being used. The processor may store, in advance, the optical parameter when the wide angle observation unit is not used and the optical parameter when the wide angle observation unit is used, and may determine the drive amount on the basis of one of the optical parameters.

The processor may acquire a deviation amount, in a depth direction along a luminous flux, between an observation target position, which is a reference for focusing the focus of the observation optical system, and an OCT target position, which is a reference for acquiring the OCT signal. The processor may drive at least one of the optical path length difference adjustment unit and the OCT focus adjustment unit in accordance with the acquired deviation amount. In this case, the adjustment of the OCT light can be rapidly and appropriately performed, regardless of whether there is the deviation between the target position of the observation optical system and the target position of the OCT unit. When the two target positions are positioned in the same location in the depth direction, the deviation amount is acquired as zero and the OCT light is adjusted.

### BRIEF DESCRIPTION OF THE DRAWINGS

Fig. 1 is a view showing a general configuration of an observation system 100.
Fig. 2 is a view showing a general configuration of an OCT unit 40.
Fig. 3 is a flowchart showing an example of observation focus automatic adjustment processing.
Fig. 4 is an explanatory diagram illustrating an interest region 81, an evaluation region 82, and a scanning position 83 that are set on a microscopic image 15.
Fig. 5 is an explanatory diagram illustrating an adjustment result of OCT light in accordance with a change in an observation focus state.
Fig. 6 is an explanatory diagram illustrating a deviation amount between an observation target position Stp and an OCT target position Otp.
Fig. 7 is a view showing an example in which there is deviation between a target position for focusing an OCT zero delay position Oz and a target position for focusing an OCT focus Of.
Fig. 8 is a flowchart showing an example of observation focus manual adjustment processing.
Fig. 9 is a view showing a general configuration of an observation system 101 when a surgical microscope 1 and the OCT unit 40 are separate devices.

### DETAILED DESCRIPTION

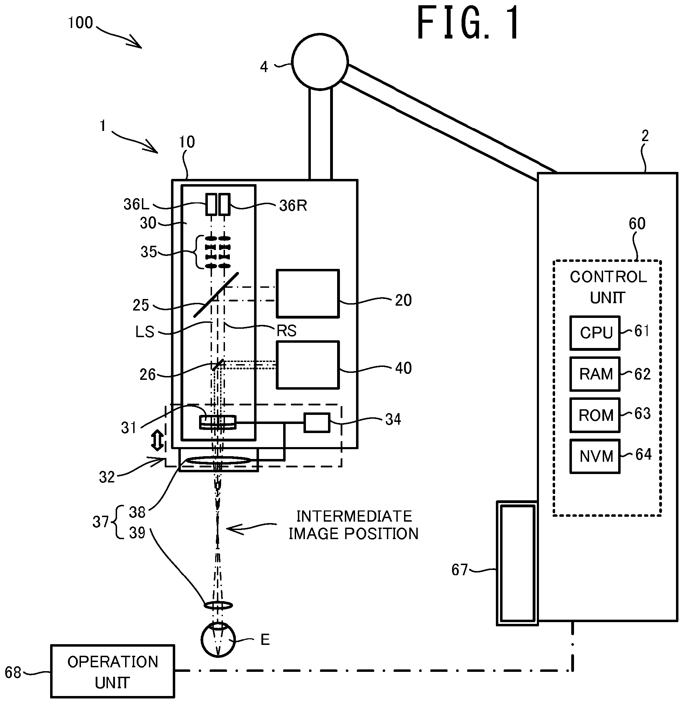

Hereinafter, an exemplary embodiment of the present disclosure will be described with reference to the drawings. The present embodiment illustrates an observation system 100 for stereoscopically viewing a patient's eye during eye surgery. However, at least a portion of the technology illustrated in the present embodiment can be applied to an observation system used for a purpose other than ophthalmology. As illustrated in FIG. 1, the observation system 100 of the present embodiment includes a surgical microscope 1, an OCT unit 40, and a control unit 60. In the following description, a direction parallel to observation luminous fluxes RS and LS of the surgical microscope 1 is referred to as a Z direction, and a direction that intersects the Z direction is referred to as an XY direction.

The surgical microscope 1 will be explained. As shown in FIG. 1, the surgical microscope 1 of the embodiment includes a base unit 2, an arm unit 4, and an observation device 10. The base unit 2 is a portion that serves as the base of the surgical microscope 1. In this embodiment, a control unit 60, which is described below, is built in the base unit 2. The arm unit 4 has at least one joint, and movably supports the observation device 10.

The observation device 10 includes an illumination optical system 20, a beam splitter 25, a reflection mirror 26, and an observation optical system 30. The illumination optical system 20 emits illumination light that illuminates a biological object (a patient's eye E in this embodiment) that is an observation object. The illumination optical system 20 is capable of emitting illumination light coaxial with the optical axis of the observation luminous flux RS for the right eye in the observation optical system 30 and illumination light coaxial with the optical axis of the observation luminous flux LS for the left eye in the observation optical system 30. However, the illumination light may be illumination light irradiated toward the observation object from an angle that differs from the optical axis of the observation luminous fluxes RS and LS. The observation luminous fluxes RS and LS in this embodiment refer to luminous fluxes guided by the observation optical system 30 to produce light to be observed by a user, of the luminous fluxes from the observation object (the luminous fluxes of the illumination light reflected by the observation object, for example).

The beam splitter 25 is one example of an optical axis coupling element that makes the optical axis of the illumination light emitted by the illumination optical system 20 and the optical axes of the observation luminous fluxes RS and LS in the observation optical system 30 coaxial. The beam splitter 25 illustrated in FIG. 1 makes the optical axis of the illumination light and the optical axes of the observation luminous fluxes RS and LS coaxial by reflecting at least a portion of the illumination light emitted from the illumination optical system 20 and transmitting at least a portion of the observation luminous fluxes RS and LS from the observation object. The illumination light reflected by the beam splitter 25 travels along the same optical path as part of the optical path of the observation luminous fluxes RS and LS, in a direction opposite to the direction in which the observation luminous fluxes RS and LS travel, and is irradiated on the observation object.

The reflection mirror 26 reflects a measurement luminous flux for measuring an OCT signal emitted by the OCT unit 40 (to be described in detail below) toward the biological object. The reflection mirror 26 illustrated in Fig. 1 is provided in a position in which there is no interference with the observation luminous fluxes RS and LS and with illumination light (in the present embodiment, a position between the two observation luminous fluxes RS and LS). In Fig. 1, the reflection mirror 26 is provided between the beam splitter 25 and an observation focus adjustment unit 32 (to be described below). However, the position of the reflection mirror 26 may be changed in accordance with a position of the OCT unit 40 or the like.

The observation optical system 30 guides the observation luminous flux from the observation object to allow the user to observe (stereoscopically view, in this embodiment) the observation object. The surgical microscope 1 in this embodiment allows the user to stereoscopically view the observation object by causing a display (a stereoscopic image display in this embodiment) 67 to display an observation image to be observed with the right eye of the user and an observation image to be observed with the left eye of the user (that is, by causing the display 67 to display left and right microscopic images). The surgical microscope 1 can thus allow the user to stereoscopically view the observation object. Therefore, the observation optical system 30 guides the right eye observation luminous flux RS from the observation object to a right eye imaging device 36R, and guides the left eye observation luminous flux LS from the observation object to a left eye imaging device 36L. The control unit 60 controls the image display of the display 67 on the basis of image signals from the two imaging devices 36R and 36L. Any of various kinds of displays, such as a three-dimensional display, a stereo viewer, and a head mounted display, for example, may be used for the display to cause stereoscopic vision of the observation object. There is no need to separately provide the right eye imaging device 36R, to which the right eye observation luminous flux RS is guided, and the left eye imaging device 36L, to which the left eye observation luminous flux LS is guided. For example, an area to which the right eye observation luminous flux RS is guided and an area to which the left eye observation luminous flux LS is guided may be provided in an imaging area of a single imaging device.

The user may observe the biological object when a wide angle observation unit 37 is used. The wide angle observation unit 37 is used to widen an observation angle of view of the ocular fundus of the patient's eye E. For example, the user may use the wide angle observation unit 37 when observing the ocular fundus of the patient's eye E. When observing the anterior ocular segment of the patient's eye E, the user may remove the wide angle observation unit 37. In this way, the user can perform an appropriate observation in accordance with each portion. The wide angle observation unit 37 of the present embodiment includes a reducing lens 38, which is arranged on the observation optical system 30 side, and a front lens 39, which is arranged on the patient's eye E side. In the present embodiment, the front lens 39 is arranged in a fixed position with respect to the patient's eye E. Thus, when the patient's eye E moves, the front lens 39 also moves. As will be described in more detail below, the control unit 60 can detect the movement of the patient's eye E by detecting the position of the front lens 39. The configuration is not limited to that described above and a configuration may be adopted in which the front lens 39 is fixed to the observation device 10.

The observation optical system 30 includes an objective lens 31, the observation focus adjustment unit 32, a zoom lens group 35, and the imaging devices 36R and 36L described above. The observation focus adjustment unit 32 is provided on optical paths of the observation luminous fluxes RS and LS. The observation focus adjustment unit 32 can adjust the focus of the observation optical system 30. The zoom lens group 35 can change an image capture magnification of an image of the biological object captured by the imaging devices 36R and 36L. In the present embodiment, the image capture magnification is changed by at least one of the lenses in the zoom lens group 35 being moved in a direction that is along the observation luminous fluxes RS and LS.

As an example, the observation focus adjustment unit 32 of the present embodiment is provided with the objective lens 31, which is a positive lens of combined lenses, and an observation focus adjustment motor 34. Further, when the wide angle observation unit 37 is used, the reducing lens (positive lens) 38 of the wide angle observation unit 37 is also included in the observation focus adjustment unit 32. The observation focus adjustment motor 34 moves the objective lens 31 (the objective lens 31 and the reducing lens 38 when the wide angle observation unit 37 is used) in a direction that is along the observation luminous fluxes RS and LS. As a result, the focus of the observation optical system 30 (hereinafter referred to as an "observation focus") is changed. In the present embodiment, as the observation focus adjustment motor 34, a motor that moves the objective lens 31 and a motor that moves the reducing lens 38 are separately provided. Thus, both the objective lens 31 and the reducing lens 38, which is arranged on the outside of a lens barrel of the objective lens 31, are appropriately moved. However, the objective lens 31 and the reducing lens 38 may be moved by a single motor.

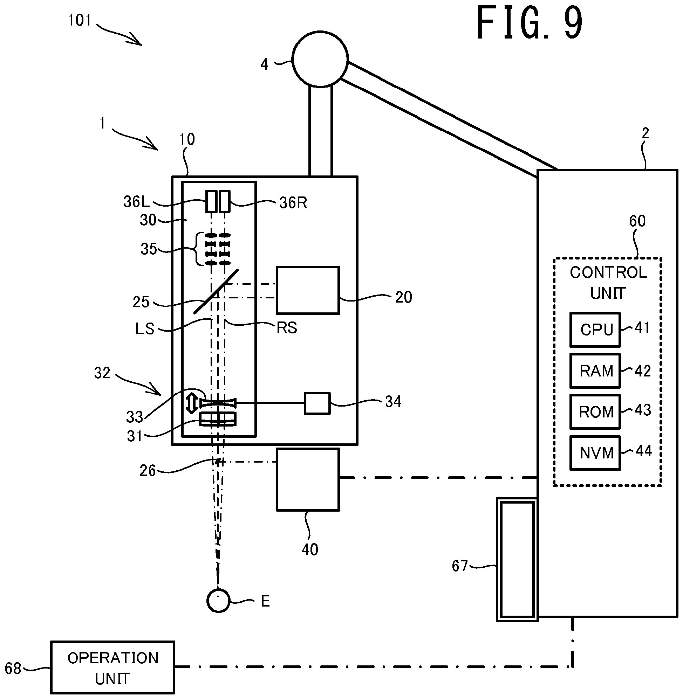

The configuration of the observation focus adjustment unit may be changed. For example, the observation focus adjustment unit may adjust the observation focus by moving at least one of the lenses in the zoom lens group 35 in a direction that is along the observation luminous fluxes RS and LS. In this case, in the example shown in Fig. 1, for example, at least one of the lenses in the zoom lens group 35 is, of the lenses arranged in order of a positive lens, a negative lens, a negative lens, and a positive lens from an upstream side on the optical path, the positive lens on the most upstream side. Further, the observation focus adjustment unit may include a negative lens 33 (see Fig. 9) that is located further to the imaging devices 36R and 36L side on the optical path than the objective lens 31. In this case, the observation focus adjustment unit may adjust the observation focus by using the observation focus adjustment motor 34 to move the negative lens 33 in a direction that is along the observation luminous fluxes RS and LS. The observation focus adjustment unit may be driven by a manual operation of the user, without using the observation focus adjustment motor.

The observation optical system 30 may include a configuration for allowing the user to stereoscopically view the observation object by looking through eyepieces. In this case, the observation optical system 30 may guide the right eye observation luminous flux RS to an eyepiece for the right eye of the user and guide the left eye observation luminous flux LS to an eyepiece for the left eye of the user.

The OCT unit 40 will be described. The OCT unit 40 uses the principles of optical coherence tomography (OCT) to acquire an OCT signal (an OCT tomographic image in the present embodiment). In the present embodiment, the OCT unit 40 is incorporated in the observation device 10 of the surgical microscope 1. In other words, in the present embodiment, the surgical microscope 1 and the OCT unit 40 are integrated. However, the surgical microscope 1 and the OCT unit 40 may be separate devices in the observation system 100.

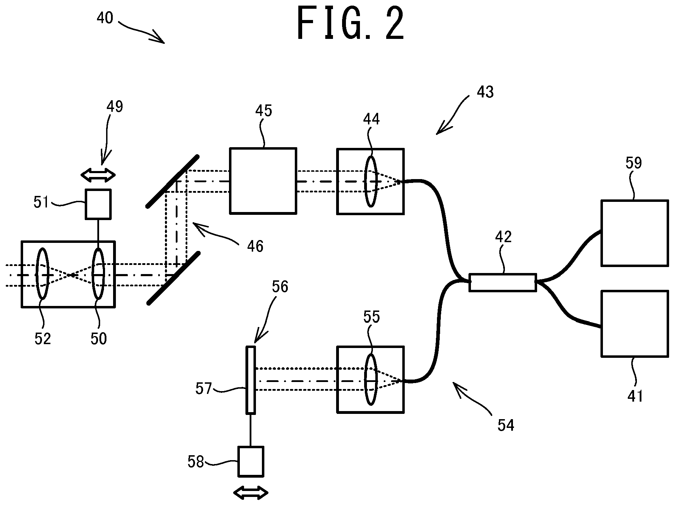

The configuration of an optical system of the OCT unit 40 will be described with reference to Fig. 2. The OCT unit 40 includes an OCT light source 41, a coupler (light splitter) 42, a measurement optical system 43, a reference optical system 54, and a photodetection element (detector) 59. The OCT light source 41 emits light (OCT light) to acquire the OCT signal. The coupler 42 splits the luminous flux emitted from the OCT light source 41 into the measurement luminous flux and a reference luminous flux. Further, the coupler 42 of the present embodiment synthesizes the measurement luminous flux reflected by the biological object and the reference luminous flux generated by the reference optical system 54, and causes the synthesized light to be received by the photodetection element 59.

The measurement optical system 43 guides the measurement luminous flux split by the coupler 42 to the biological object (the patient's eye E), and returns the measurement luminous flux reflected by the biological object to the coupler 42. In the present embodiment, the measurement optical system 43 includes a collimator lens 44, an NA adjustment unit 45, an optical scanner 46, a lens 50, and a lens 52, in that order from the upstream side (the OCT light source 41 side) of the optical path. The collimator lens 44 causes the measurement luminous flux that has been split by the coupler 42 and has passed through a fiber to be a parallel luminous flux.

By changing a beam diameter of the measurement luminous flux injected as the parallel luminous flux from the collimator lens 44, the NA adjustment unit 45 adjusts a numerical aperture NA of the measurement luminous flux concentrated toward the biological object. As an example, a known infinity focus zoom system is adopted in the NA adjustment unit 45 of the present embodiment. The control unit 60 controls the drive of a motor (not illustrated) provided in the NA adjustment unit 45 to move a lens provided in the NA adjustment unit 45 in an optical axis direction, thus changing the beam diameter. As a result, the numerical aperture NA of the measurement luminous flux concentrated toward the biological object is adjusted, and a horizontal resolution capacity and a focal depth at the time of acquiring the OCT signal are adjusted.

The optical scanner 46 causes the measurement luminous flux to scan in a two-dimensional direction as a result of being driven by a drive unit (not illustrated). As a result, an acquisition position of the OCT signal on the biological object is determined. The optical scanner 46 of the present embodiment is provided in a position substantially conjugate to the pupil of the patient's eye E. Further, in the present embodiment, two galvanometer mirrors, which can deflect the measurement luminous flux in mutually different directions, are used as the optical scanner 46. However, another device that deflects the light (at least one of a polygon mirror, a resonant scanner, an acousto-optic device, and the like) may be used as the optical scanner 46.

The lens 50 and the lens 52 are provided further to a downstream side (namely, the biological object side) on the optical path of the measurement luminous flux than the optical scanner 46. The lens 50 and the lens 52 function as a projection optical system that projects the measurement luminous flux toward the patient's eye E. The lenses 50 and 52 of the present embodiment are used in a Keplerian telescope. The OCT unit 40 includes an OCT focus adjustment unit 49 that adjusts the focus (hereinafter referred to as the "OCT focus") of the measurement optical system 43 that optically guides the measurement luminous flux. The OCT focus adjustment unit 49 illustrated in Fig. 2 includes the lens 50 of the front group of the Keplerian telescope, and an OCT focus adjustment motor 51. The OCT focus adjustment motor 51 can adjust the OCT focus by moving the lens 50 in a direction that is along the measurement luminous flux.

The reference optical system 54 optically guides the reference luminous flux and returns the reference luminous flux to the coupler 42. The reference optical system 54 of the present embodiment includes a collimator lens 55 and a reference mirror 57. The collimator lens 55 causes the reference luminous flux that has been split by the coupler 42 and has passed through a fiber to be a parallel luminous flux. The reference mirror 57 reflects the reference luminous flux and returns the reference luminous flux to the coupler 42. The configuration of the reference optical system 54 may be changed. For example, the reference optical system 54 may return the reference luminous flux that has been optically guided from the coupler 42 to the coupler 42 using a transmission type optical system, such as an optical fiber, without reflecting the reference luminous flux.

The OCT unit 40 includes an optical path length difference adjustment unit 56 that adjusts the optical path length difference between the measurement luminous flux and the reference luminous flux. By adjusting the optical path length difference, a range in a depth direction (the Z direction) over which the OCT signal is acquired is changed. The range in the depth direction over which the OCT signal is acquired is, for example, a field of view in the depth direction of the tomographic image, when the tomographic image is acquired. The optical path length difference adjustment unit 56 is provided on at least one of the optical path of the measurement luminous flux and the optical path of the reference luminous flux. The optical path length difference adjustment unit 56 illustrated in Fig. 2 includes the reference mirror 57 and an optical path length difference adjustment motor 58. The optical path length difference adjustment motor 58 adjusts the optical path length difference by moving the reference mirror 57 in a direction that is along the reference luminous flux. The configuration of the optical path length difference adjustment unit may be changed as appropriate. For example, the optical path length difference adjustment unit may adjust the optical path length difference by moving, in an optical axis direction, the collimator lens 44 provided on the optical path of the measurement luminous flux, and an end portion of the fiber that guides the measurement luminous flux from the coupler 42. Further, the optical path length difference adjustment unit may adjust the optical path length difference by moving, in the optical axis direction, the collimator lens 55 provided on the optical path of the reference luminous flux, and an end portion of the fiber that guides the reference luminous flux from the coupler 42.

The photodetection element 59 detects an interference state of the measurement luminous flux and the reference luminous flux. In the case of Fourierdomain OCT, the spectral intensity of an interference light is detected by the photodetection element 59, and a depth profile (an A scan signal) of a specified range is acquired by applying a Fourier transform to spectral intensity data. Various types of OCT can be used in the observation system 100. For example, any of spectral-domain OCT (SD-OCT), swept-source OCT (SS-OCT), time-domain OCT (TD-OCT), and the like may be adopted in the observation system 100.

Returning now to the description of FIG. 1, the control unit 60 controls various operations of the observation system 100 (such as adjustment control of the OCT light by the OCT unit 40). The control unit 60 includes a CPU 61, a RAM 62, a ROM 63, and a non-volatile memory (NVM) 64. The CPU 61 is a controller that performs various types of control. The RAM 62 temporarily stores various kinds of information. The ROM 63 stores programs to be executed by the CPU 61, various initial values, and the like. The NVM 64 is a non-transitory storage medium capable of retaining stored content even when the supply of power is cut off. The NVM 64 may store an observation control program for executing various types of processing, which is described below.

In the present embodiment, as an example, the control unit 60 provided in the surgical microscope 1 functions as the control unit that performs the adjustment control of the OCT light and the like. Specifically, in the present embodiment, the surgical microscope 1 functions as the control device that controls the observation system 100. However, the configuration of the control unit that controls the observation system 100 (namely, the control unit that executes an observation control program) may be changed as appropriate. For example, the OCT device that is provided as the separate device to the surgical microscope 1 may function as the control device that controls the observation system 100. In addition, a control unit of a personal computer (not shown) connected to the surgical microscope 1 may control the observation system 100. Control units provided in each of a plurality of devices (the control unit 60 of the surgical microscope 1 and the control unit of the OCT device, for example) may operate in concert to control the observation system 100.

The operation unit 68 may be operated by the user to input various operation commands into the observation system 100. In this embodiment, a foot switch, which is operated by a foot of the user, is at least provided as the operation unit 68. Therefore, the user can input various operation commands from the foot switch 68, while handling a surgical instrument with the user's hand. However, another device (various buttons or a touch panel, for example) may be used as the operation unit 68, together with the foot switch, or instead of the foot switch.

### Acquiring focus state of observation optical system

The control unit 60 of the present embodiment can acquire a focus state of the observation optical system 30 of the surgical microscope 1 (hereinafter also simply referred to as an "observation focus state"). An example of a method for acquiring the observation focus state will be described below. The control unit 60 of the present embodiment acquires the observation focus state on the basis of a signal from the imaging devices 36R and 36L. Thus, in the observation system 100 of the present embodiment, it is possible to omit a configuration dedicated to acquiring (detecting) the observation focus state.

As an example, the control unit 60 of the present embodiment acquires the observation focus state using a contrast detection method or an image surface phase-difference detection method. In the contrast detection method, the control unit 60 analyzes a microscopic image captured by the imaging devices 36R and 36L, while changing the observation focus using the observation focus adjustment unit 32, and detects a contrast of the microscopic image. The control unit 60 acquires the observation focus state by causing a position at which the contrast of the microscopic image is the highest to be the position at which the observation focus is in focus. Further, in the image surface phase-difference detection method, a phase difference pixel is embedded among pixels of at least one of the imaging devices 36R and 36L. The phase difference pixel is formed in a non-symmetrical shape in the left-right direction to detect a phase difference (parallax) of the image. The control unit 60 calculates the phase difference on the basis of a signal obtained by light injected from one of the left and right directions being selectively received by the phase difference pixel. The control unit 60 acquires the observation focus state by causing a position at which the phase difference is smaller to be the position at which the observation focus is in focus.

As described above, as the method to acquire the observation focus state, a method can be used that does not make use of the signal from the imaging devices 36R and 36L. In this case, the surgical microscope 1 may have a configuration that enables the user to observe the biological object via an ocular lens, without being provided with the imaging devices 36R and 36L.

### Observation system control processing

Below, observation system control processing performed by the control unit of the observation system 100 (the control unit 60 of the surgical microscope 1 in the present embodiment) will be described. The CPU 61 of the control unit 60 performs various types of processing to be described below, in accordance with the observation system control program stored in the NVM 64.

### Observation focus automatic adjustment processing

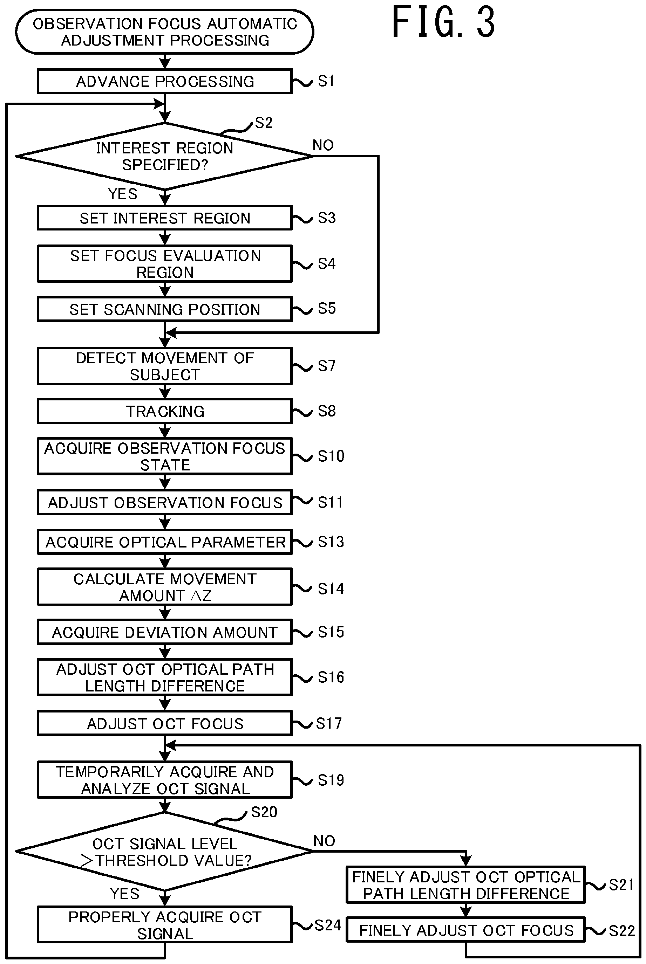

Observation focus automatic adjustment processing will be described with reference to Fig. 3 to Fig. 7. The observation focus adjustment processing is an example of processing performed when automatically adjusting the observation focus.

As illustrated in Fig. 3, the CPU 61 starts advance processing (S1). As described above, the CPU 61 captures an image of the patient's eye E using the imaging devices 36R and 36L of the observation optical system 30. In the advance processing, the CPU 61 starts display of the microscopic image on the display 67, on the basis of the imaging signal from the imaging devices 36R and 36L. Further, the CPU 61 adjusts the optical path length difference in the OCT unit 40 on the basis of an optical parameter of the observation optical system 30. For example, a case is illustrated in which the objective lens 31 whose focal length can be changed is used and the focal length is changed from 170 mm to 200 mm. In this case, a distance from the objective lens 31 to the patient's eye E (a working distance) becomes approximately 30 mm longer. Thus, the CPU 61 controls the driving of the optical path length difference adjustment unit 56 on the basis of the optical parameter (the focal length) of the observation optical system 30, and causes the optical path length difference to change by approximately 30 mm.

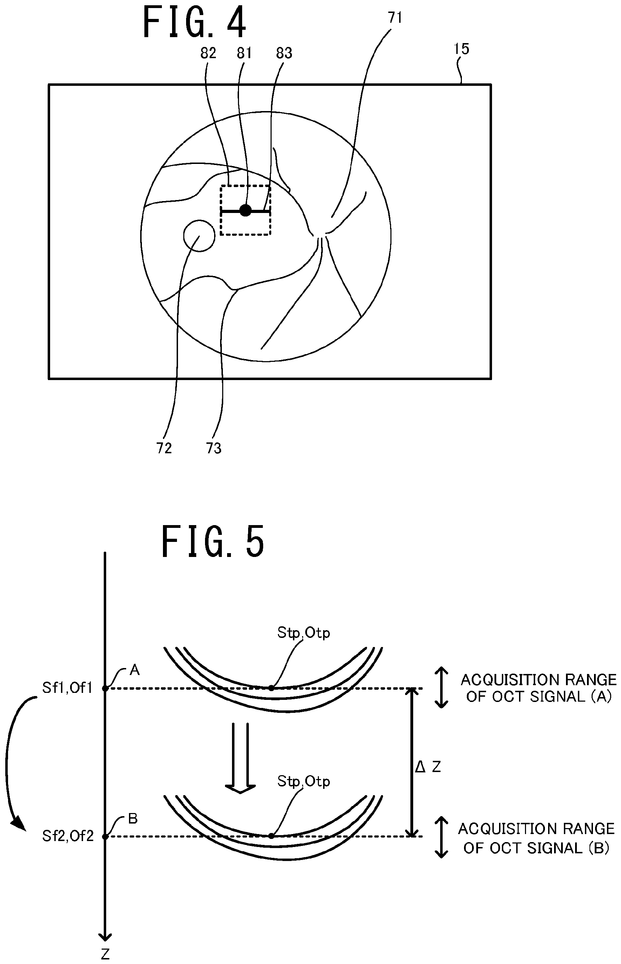

Next, the CPU 61 determines whether a command specifying an interest region has been input by the user (S2). The interest region is a region (including a region of a point), inside an image region of the microscopic image, that is used as a reference for adjusting the focus of the observation optical system 30. In the present embodiment, as illustrated in Fig. 4, the user may input the command specifying an interest region 81, by operating the operation unit 68 while viewing a microscopic image 15 displayed on the display 67. Thus, when a region to which the user wishes to pay particular attention is present on the microscopic image 15, the user may specify the region to which the user wishes to pay attention as the interest region 81, and may observe the microscopic image 15 in a state in which the region to which the user wishes to pay attention is in focus with regard to the observation focus. Further, in the present embodiment, the user may change the interest region 81 by operating the operation unit 68 during observation. When the interest region 81 is specified (YES at S2), the CPU 61 sets the interest region 81 as the position specified within the image region of the microscopic image 15 (S3). The specification of the interest region 81 includes a specification to change the interest region 81.

Next, the CPU 61 sets a focus evaluation region 82 within the image region of the microscopic image 15 (S4). The focus evaluation region (hereinafter also simply referred to as "evaluation region") 82 is a region that includes the set interest region 81. As described above, in the present embodiment, the observation focus state is acquired on the basis of the signal from the imaging devices 36R and 36L that capture the microscopic image 15. The evaluation region 82 is a region, in the microscopic image 15, for which the signal for acquiring the observation focus state is acquired from the imaging devices 36R and 36L. As will be described in more detail below, when the evaluation region 82 is set, the CPU 61 acquires the observation focus state on the basis of the signal from the imaging devices 36R and 36L for the evaluation region 82, and adjusts the observation focus (S10 and S11). Further, as shown in Fig. 4, the interest region 81 is included in the evaluation region 82 set at S4. Thus, the microscopic image 15 is captured in a state in which the observation focus is focused on the interest region 81.

Next, on the basis of at least one of the interest region 81 and the evaluation region 82 that are set, the CPU 61 sets a scanning position 83 of the measurement luminous flux to acquire the OCT signal (S5). In this case, the scanning position 83 is linked to at least one of the interest region 81 and the evaluation region 82. Thus, the user may use the high quality microscopic image 15 and the high quality OCT image to observe the position to which the user wishes to pay attention. In the example illustrated in Fig. 4, the CPU 61 sets the scanning position 83 to pass through the interest region 81 and to be contained within the evaluation region 82. However, the method for setting the scanning position 83 may be changed as appropriate. For example, the scanning position 83 may extend to the outside of the evaluation region 82. The CPU 61 may set the scanning position 83 within the evaluation region 82 irrespective of the interest region 81.

If the command specifying the interest region 81 has not been input (NO at S2), the processing moves to S7 without performing the processing at S3 to S5. The method for setting the interest region 81 and the evaluation region 82 may be changed. For example, by performing image processing on the microscopic image 15 and detecting a particular portion of the biological object included in the microscopic image 15 (at least one of an optic disc 71, a macula lutea 72, and a fundus oculi blood vessel 73 shown in Fig. 4, for example), the CPU 61 may set the interest region 81 at a position having a specified relationship to the particular portion. A particular region within the captured image region (a center of the image region, for example) may be set in advance as the interest region 81. The whole of the image region of the microscopic image 15 may be set as the evaluation region 82. The scanning position 83 may be set independently of the interest region 81 and the evaluation region 82. The CPU 61 may set at least one of the interest region 81 and the evaluation region 82 on the basis of the specified scanning position 83, after the scanning position 83 of the OCT measurement luminous flux has been specified by the user. For example, the CPU 61 may set a region including the scanning position 83 as the evaluation region 82.

Next, by performing image processing on the microscopic image 15 captured by the imaging devices 36R and 36L, the CPU 61 detects a movement of a subject captured in the microscopic image 15 (S7). For example, the CPU 61 may perform the image processing to detect the particular portion of the biological object (at least one of the optic disc 71, the macula lutea 72, and the fundus oculi blood vessel 73 shown in Fig. 4, for example), and may detect a movement of the particular portion to detect the movement of the subject. When the wide angle observation unit 37 (see Fig. 1) is being used, the CPU 61 may detect the movement of the subject by detecting a movement of the position of the front lens 39 provided in the wide angle observation unit 37 (a position of an opening of the front lens 39, for example).

Next, in accordance with the detected movement of the subject, the CPU 61 causes the position of the evaluation region 82 in the microscopic image 15 to be tracked (performs tracking) (S8). As a result, even if the subject moves, the quality of the microscopic image 15 in the interest region 81 is favorably maintained. The CPU 61 also performs processing that causes the scanning position 83 of the OCT measurement luminous flux to be tracked (performs tracking) in accordance with the detected movement of the subject.

Next, the CPU 61 acquires the observation focus state (S10). As described above, the CPU 61 of the present embodiment acquires the observation focus state on the basis of the signal from the imaging devices 36R and 36L for the evaluation region 82. However, the CPU 61 can acquire the observation focus state on the basis of the signal for the whole of the microscopic image 15. The observation focus state may be acquired without using the signal from the imaging devices 36R and 36L.

Next, on the basis of an acquisition result of the observation focus state, the CPU 61 adjusts the observation focus by controlling the drive of the observation focus adjustment unit 32 (S11). In other words, the CPU 61 performs automatic focusing of the observation optical system 30. In the example shown in Fig. 5, for example, an observation target position Stp, which is a reference for focusing the observation focus, is set on the retina of the ocular fundus. In the present embodiment, the observation target position Stp matches the interest region 81. As long as the patient's eye E does not move, the observation focus in the Z direction is maintained as Sfl, which is focused on the observation target position Stp. Here, when the patient's eye E moves in the positive Z direction (downward in Fig. 5), the CPU 61 detects that the observation focus state is out of focus. Then, the CPU 61 changes the observation focus in the Z direction to Sf2, such that the observation focus is brought into focus on the observation target position Stp that has moved in the + Z direction. Also, in a case in which the observation target position Stp is changed by a command of the user or the like, the appropriate observation focus may be changed. In this case also, in the present embodiment, the observation focus is appropriately adjusted.

Next, processing to adjust the OCT light in accordance with the changes in the observation focus state is performed (S13 to S17). As an example, on the basis of the optical parameter of the observation optical system 30 (including the observation focus adjustment unit 32) and a drive amount of the observation focus adjustment unit 32, the CPU 61 of the present embodiment calculates an amount ΔZ by which an object position at which the observation focus is to be focused has moved. The object position at which the observation focus is to be focused is, for example, the observation target position Stp on the ocular fundus in the example shown in Fig. 5. The CPU 61 adjusts the OCT light on the basis of the movement amount ΔZ. This will be described in more detail below.

First, the CPU 61 acquires the optical parameter of the observation optical system 30 (S13). In the present embodiment, the focal length of the observation optical system 30 that is changed by the observation focus adjustment unit 32 is acquired. Further, in the present embodiment, the optical parameter changes depending on whether the wide angle observation unit 37 (see Fig. 1) is used. Thus, the CPU 61 of the present embodiment acquires the optical parameter in accordance with the presence or absence of the wide angle observation unit 37. In a specific example, in the present embodiment, the CPU 61 acquires, as the optical parameters of the observation optical system 30, an image formation magnification β₁ and a longitudinal magnification α₁ for the observation object in relation to an intermediate image plane of the observation optical system 30 (a front side focal position of the front lens 39) (, which change depending on whether the wide angle observation unit 37 is used and in accordance with the focal length). Further, in the present embodiment, the CPU 61 acquires a focal position of the lens 50 of the front group of the Keplerian telescope of the OCT unit 40 with respect to the observation object, namely, acquires an imaging magnification β₂ and a longitudinal magnification α₂ for an intermediate image of an end face of the fiber.

Next, on the basis of the change in the observation focus state, the CPU 61 calculates the amount ΔZ by which the object position at which the observation focus is to be focused has moved in the Z direction (S14). For example, in the example shown in Fig. 5, the observation focus is adjusted by the observation focus adjustment unit 32 as a result of the observation target position Stp, at which the observation focus is to be focused, moving in the positive Z direction. Thus, the CPU 61 can calculate the movement amount ΔZ of the object position, on the basis of the drive amount of the observation focus adjustment unit 32. In the example shown in Fig. 5, if the movement amount of the observation focus with respect to the position of the intermediate image is denoted by ΔZ₁, then ΔZ = α₁ × ΔZ₁ = β₁² × ΔZ₁ is obtained. The movement amount of the observation focus with respect to the position of the intermediate image matches an extending amount of the objective lens 31 and the reducing lens 38 of the observation focus adjustment unit 32.

Next, the CPU 61 acquires a deviation amount, in a depth direction along the luminous flux, between the observation target position, which is the reference for focusing an observation focus Sf, and an OCT target position, which is a reference for acquiring the OCT signal (S15). The OCT target position is at least one of a target position, which is a reference for focusing a zero delay position (a position at which the optical path length difference of the OCT light is zero), and a target position, which is a reference for focusing an OCT focus Of. In the observation system 100 of the present embodiment, the user may independently specify the observation target position and the OCT target position by operating the operation portion 68. When the observation target position and the OCT target position are aligned in the depth direction (as in Fig. 5, for example), the deviation amount acquired at S15 is zero.

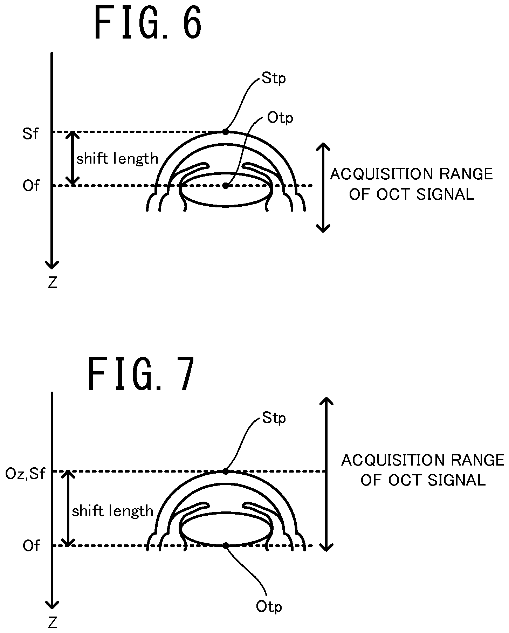

In contrast to Fig. 5, in Fig. 6 and Fig. 7, a case is illustrated in which the ocular fundus of the patient's eye E is not observed and an anterior ocular segment thereof is observed. In the example shown in Fig. 6, the observation target position Stp is set to the corneal apex of the patient's eye E. With respect to this, an OCT target position Otp, which is the reference for focusing both the zero delay position and the OCT focus, is set to the interior of the crystalline lens of the eye. In this case, the deviation amount between the observation target position Stp and the OCT target position Otp is "shift length" shown in the figures.

Further, in an example shown in Fig. 7, there is a deviation in the Z direction between a target position for focusing a zero delay position Oz of the OCT and the target position Otp for focusing the OCT focus Of. In this way, the zero delay position Oz and the OCT focus Of can be set so as to deviate from each other. In the example shown in Fig. 7, the target position for focusing the zero delay position Oz and the observation target position Stp are both set to the corneal apex. The target position Otp for focusing the OCT focus Of is set to the rear surface of the crystalline lens. As a result of this, the favorable OCT signal can be acquired over a wide range from the corneal apex to the rear surface of the crystalline lens. In the example shown in Fig. 7, the CPU 61 acquires the deviation amount "shift length" between the observation target position Stp and the target position Otp for focusing the OCT focus Of at S15.

Next, by driving the optical path length difference adjustment unit 56 of the OCT unit 40, on the basis of the movement amount ΔZ of the object position, the CPU 61 adjusts the optical path length difference (S16). In the present embodiment, the CPU 61 controls the drive of the optical path length difference adjustment motor 58 of the optical path length difference adjustment unit 56 to move the reference mirror 57 by ΔZ × n_{g} (where n_{g} is a group index of the patient's eye E), thus adjusting the optical path length difference. For example, in the example shown in Fig. 5, the optical path length difference is adjusted in accordance with the change of the observation focus from Sf1 to Sf2. As a result of this, an acquisition range of the OCT signal centered on a point A in the Z direction is changed to an acquisition range of the OCT signal centered on a point B. When there is the deviation between the observation target position Stp and the target position for focusing the zero delay position (see Fig. 6, for example), the CPU 61 drives the optical path length difference adjustment unit 56 while taking into account the deviation amount acquired at S15. Thus, even when there is deviation between the observation target position Stp and the target position for focusing the zero delay position, the optical path length difference can be appropriately adjusted.

Next, by driving the OCT focus adjustment unit 49 on the basis of the movement amount ΔZ of the object position, the CPU 61 adjusts the OCT focus (S17). In the present embodiment, as shown in Fig. 1, the measurement luminous flux of the OCT also passes through the observation focus adjustment unit 32. Thus, when the observation focus adjustment unit 32 is driven, the OCT focus also moves. However, due to a wavelength difference and the like between the observation light of the surgical microscope 1 and the OCT light, there may be a case in which the movement amount of the observation focus and the movement amount of the OCT focus in relation to the drive amount of the observation focus adjustment unit 32 do not match each other. Thus, the CPU 61 of the present embodiment more accurately adjusts the OCT focus by also driving the OCT focus adjustment unit 49 in accordance with the changes in the observation focus state.

The drive amount of the OCT focus adjustment unit 49 can be calculated on the basis of an optical parameter of the measurement optical system 43 and the like. As an example, if the movement amount of the OCT focus at the intermediate image position is denoted by ΔZ₂, the image formation magnification of the measurement optical system 43 is denoted by β₂, the longitudinal magnification is denoted by α₂, and the drive amount of the OCT focus adjustment unit 49 (the movement amount of the lens 52 in the present embodiment) is denoted by ΔZ₃, then ΔZ₂ =α₂ × ΔZ₃ = β₂² × ΔZ₃ is obtained. ΔZ₂ is determined in accordance with the movement amount ΔZ of the object position that is calculated at S14, and thus, from the above-described formula, the drive amount of the OCT focus adjustment unit 49 can be calculated. When there is the deviation between the observation target position Stp and the target position for focusing the OCT focus Of (as in Fig. 6 and Fig. 7, for example), the CPU 61 drives the OCT focus adjustment unit 49 while also taking into account the deviation amount acquired at S15.

By the processing from S13 to S17 being performed as described above, the adjustment of the OCT light is performed in accordance with the change in the observation focus state. As a result, the optical path length difference and the OCT focus of the OCT unit 40 can be rapidly and easily adjusted. Here, the OCT light may be adjusted only in accordance with the change in the observation focus state, but the CPU 61 of the present embodiment also adjusts the OCT light on the basis of an analysis result of the OCT signal (S19 to S22). As a result, the adjustment of the OCT light can be more appropriately adjusted. This will be described in detail below.

First, the CPU 61 temporarily acquires the OCT signal via the photodetection element 59 and analyzes the OCT signal (S19). Next, the CPU 61 determines whether the level of the analyzed OCT signal is greater than a threshold value (S20). When the level of the OCT signal is equal to or less than the threshold value (NO at S20), the CPU 61 drives the optical path length difference adjustment unit 56 to finely adjust the optical path length difference (S21). Further, the CPU 61 drives the OCT focus adjustment unit 49 to finely adjust the OCT focus (S22). The processing returns to S19 and the processing from S19 to S22 is repeated until the level of the OCT signal is greater than the threshold value. Since the optical path length difference and the OCT focus are generally appropriately adjusted in accordance with the changes in the observation focus state, a range of the adjustment on the basis of the analysis result of the OCT signal (S19 to S22) can be made narrower. Thus, the OCT light can be rapidly and appropriately adjusted. When the level of the OCT signal becomes greater than the threshold value (YES at S20), the CPU 61 properly acquires the OCT signal (S24). The processing then returns to the determination at S2.

The method for analyzing the OCT signal may be selected as appropriate. For example, the CPU 61 may analyze signals for the whole of the acquired OCT image. Alternatively, of the acquired OCT image, the CPU 61 may analyze a signal for a specified range centered on the OCT target position Otp. In this case, the quality of the OCT image in the range that includes the OCT target position Otp can be further improved. Of the acquired OCT image, the CPU 61 may carry out weighting such that the analysis result in the vicinity of the OCT target position Otp is accorded more significance than an analysis result of a position separated from the Otp.

In the observation focus automatic adjustment processing shown in Fig. 3, the automatic focusing of the observation optical system 30 is constantly performed during the observation of the biological object using the surgical microscope 1. However, the method for performing the automatic focusing may be changed. For example, the CPU 61 may acquire the focus state of the observation optical system 30 and perform the automatic focusing when a command to perform the automatic focusing has been input by the user. The automatic focusing may be switched on and off in accordance with a command of the user.

Observation focus manual adjustment processing In the observation focus automatic adjustment processing illustrated in Fig. 3 to Fig. 7, in addition to the observation focus being automatically adjusted, the OCT light is also automatically adjusted in accordance with the change in the observation focus state. However, when the observation focus is adjusted by a manual operation of the user (including an operation using a foot switch), at least a part of the technology illustrated by the above-described embodiment may be applied.

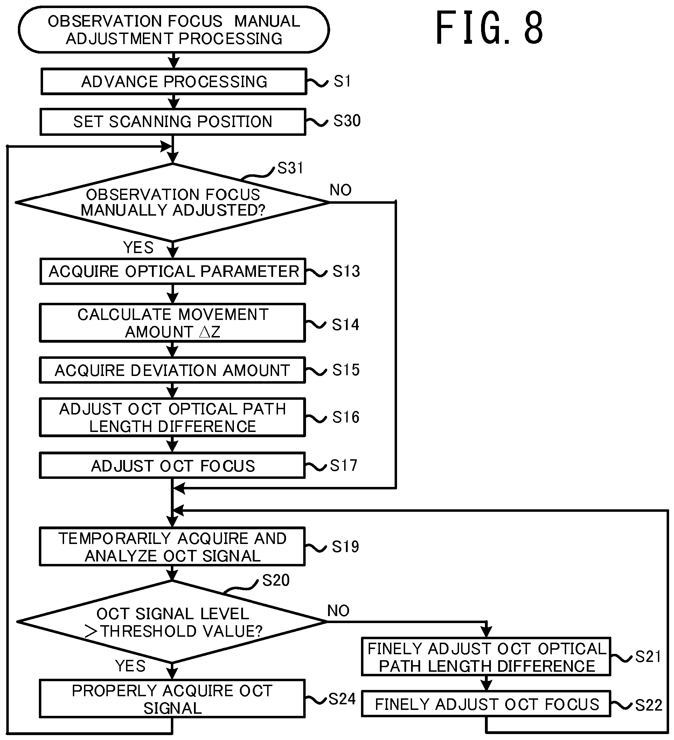

Observation focus manual adjustment processing will be described with reference to Fig. 8. The observation focus manual adjustment processing is an example of processing performed when the observation focus is adjusted by the manual operation of the user. In part of the processing shown in Fig. 8, the same processing as that of the observation focus automatic adjustment processing shown in Fig. 3 can be applied. Thus, steps for which the same processing as the processing shown in Fig. 3 can be applied are assigned with the same step number as shown in Fig. 3, and an explanation thereof is omitted or simplified.

As shown in Fig. 8, the CPU 61 performs the advance processing (S1), and sets the scanning position of the OCT measurement luminous flux (S30). In the processing shown in Fig. 8, the CPU 61 sets the scanning position on the microscopic image in accordance with a command input on the operation portion 68 that specifies the scanning position.

Next, the CPU 61 determines whether the observation focus has been manually adjusted (S31). In the example shown in Fig. 8, the user may input an observation focus adjustment command, by operating the operation portion 68 while viewing the microscopic image displayed on the display 67. The CPU 61 performs the observation focus adjustment in accordance with the command, by driving the observation focus adjustment motor 34 in accordance with the command input by the user. However, the method for manually adjusting the observation focus may be changed. For example, in place of the observation focus adjustment motor 34, the surgical microscope 1 may be provided with a configuration in which the observation focus is adjusted by manually rotating a lens barrel of a lens or so on. In this case, the CPU 61 may acquire the change in the observation focus state on the basis of a signal from a potentiometer or the like that is provided in an observation focus adjustment unit.

When it is determined that the observation focus has been manually adjusted (YES at S31), the OCT light is adjusted in accordance with the change in the observation focus state (namely, the change in the observation focus due to a manual operation) (S13 to S17). When the observation focus adjustment has not been manually performed (NO at S31), only the adjustment of the OCT light on the basis of the analysis result of the OCT signal is performed (S19 to S22).

### Changes to OCT unit

In the observation system 100 illustrated in Fig. 1, the surgical microscope 1 and the OCT unit 40 are configured in an integrated manner. However, as in an observation system 101 shown in Fig. 9, the OCT unit 40 may be a device that is separate from the surgical microscope 1. In this case, the OCT unit 40 is connected by a communication link to the control unit 60.

Further, in the observation system 100 illustrated in Fig. 1, the reflection mirror 26 that reflects the OCT measurement luminous flux is provided further to the downstream side of the observation luminous fluxes RS and LS (the side of the imaging devices 36R and 36L) than the observation focus adjustment unit 32. Thus, when the observation focus adjustment unit 32 is driven, the OCT focus changes. However, the position of the reflection mirror 26 may be changed. For example, as in the observation system 101 shown in Fig. 9, the reflection mirror 26 may be provided further to the side of the biological object than the observation focus adjustment unit 32. In this case, the OCT focus is adjusted independently of the observation focus. Thus, as illustrated in Fig. 3 and Fig. 8, it is preferable that the CPU 61 perform the adjustment of the optical path length difference (S16) and the adjustment of the OCT focus (S17), in accordance with the change in the observation focus state.

The technology disclosed in the above-described embodiment is merely an example. Thus, the technology illustrated in the above-described embodiment may be modified. For example, in the embodiment illustrated in Fig. 1 to Fig. 7, the adjustment of the optical path length difference (S16) and the adjustment of the OCT focus (S17) are performed in accordance with the change in the observation focus state. As a result, the OCT focus can be rapidly and appropriately adjusted. However, in the example shown in Fig. 1, the OCT focus changes along with the observation focus by the driving of the observation focus adjustment unit 32. Therefore, in a case in which a difference between the movement amount of the observation focus and the movement amount of the OCT focus is small and the like when the observation focus adjustment unit 32 is driven, it is possible to perform the adjustment of the optical path length difference in accordance with the change in the observation focus state (S16), while omitting the drive processing of the OCT focus adjustment unit 49 in accordance with the change in the observation focus state (S17).

Further, a determination may be made, on the basis of another condition, whether to perform the drive processing of the OCT focus adjustment unit 49 in accordance with the change in the observation focus state (S17). For example, when using the OCT unit 40 to capture a tomographic image of an anterior ocular segment of the patient's eye E or the like, in order to prioritize deepening of the focal depth rather than enhancing the resolution, there may be a case in which the numerical aperture NA is made smaller by the NA adjustment unit 45. In contrast, in the case of capturing a tomographic image of the ocular fundus of the patient's eye E or the like, in order to prioritize the enhancing of the resolution, there may be a case in which the numerical aperture NA is made larger by the NA adjustment unit 45. When the numerical aperture NA is made larger, the focal depth becomes shallower and it is thus preferable for the OCT focus to be more accurately adjusted by the OCT focus adjustment unit 49. Therefore, the CPU 61 may determine, on the basis of the numerical aperture NA adjusted by the NA adjustment unit, whether to perform the drive processing of the OCT focus adjustment unit 49 in accordance with the change in the observation focus state (S17). For example, the CPU 61 may determine whether to perform the processing at S17 depending on whether the numerical aperture NA is equal to or greater than a threshold value.

The CPU 61 may omit the adjustment of the optical path length difference in accordance with the change in the observation focus state (S16) and may perform the drive processing of the OCT focus adjustment unit 49 in accordance with the change in the observation focus state (S17).

In the above-described embodiment, the fine adjustment of the OCT optical path length difference (S21) and the fine adjustment of the OCT focus (S22) are both performed in accordance with the analysis result of the OCT signal. However, the processing at least one of S21 or S22 may be omitted.

## Claims

1. An observation system (100, 101) comprising:
a surgical microscope (1);
an OCT unit (40) adapted to acquire an OCT signal; and
a processor (61),
wherein
the surgical microscope includes:
an observation optical system (30) adapted to optically guide an observation luminous flux from a biological object being an observation object; and
an observation focus adjustment unit (32) provided on an optical path of the observation luminous flux in the observation optical system, the observation focus adjustment unit being adapted to adjust a focus of the observation optical system,
the OCT unit includes:
an OCT light source (41);
a light splitter (42) adapted to split a luminous flux emitted from the OCT light source into a measurement luminous flux and a reference luminous flux;
a photodetection element (59) adapted to receive an interference light obtained by synthesis of the reference luminous flux and a measurement luminous flux reflected by the biological object; and
an optical path length difference adjustment unit (56) provided on at least one of an optical path of the measurement luminous flux and an optical path of the reference luminous flux, the optical path length difference adjustment unit being adapted to adjust an optical path length difference between the measurement luminous flux and the reference luminous flux,
the processor is adapted to acquire a focus state of the observation optical system and to drive the optical path length difference adjustment unit of the OCT unit in accordance with a change in a focus state of the observation optical system,
the OCT unit further includes:
an OCT focus adjustment unit (49) provided on the optical path of the measurement luminous flux, the OCT focus adjustment unit being adapted to adjust a focus of an optical system that optically guides the measurement luminous flux, **characterised in that**
the processor is adapted to drive the OCT focus adjustment unit in accordance with a change in the focus state of the observation optical system.

2. The observation system according to claim 1, wherein
the processor is adapted to:
acquire the focus state of the observation optical system of the surgical microscope; and
drive the observation focus adjustment unit and the optical path length difference adjustment unit on the basis of an acquisition result of the focus state of the observation optical system.

3. The observation system according to claim 2, wherein
the surgical microscope further includes an imaging device (36R, 36L) adapted to capture a microscopic image of the biological object by receiving the observation luminous flux optically guided by the observation optical system, and
the processor is adapted to:
cause a display (67) to display the microscopic image captured by the imaging device; and
acquire the focus state of the observation optical system on the basis of a signal from the imaging device.

4. The observation system according to claim 3, wherein
the processor is adapted to:
set an interest region (81) within a region of the microscopic image captured by the imaging device; and
acquire the focus state of the observation optical system on the basis of a signal for an evaluation region (82), the evaluation region being a region, of the microscopic image, that includes the set interest region.

5. The observation system according to claim 4, wherein
the processor is adapted to set a position (83) at which the OCT unit causes the measurement luminous flux to scan, on the basis of at least one of the interest region and the evaluation region.

6. The observation system according to either one of claims 4 and 5, wherein
the processor is adapted to:
detect a movement of a subject captured in the microscopic image by performing image processing on the microscopic image captured by the imaging device; and
cause a position of the evaluation region in the microscopic image to be tracked in accordance with the detected movement of the subject.

7. The observation system according to any one of claims 1 to 6, wherein
the processor is adapted to:
analyze an OCT signal acquired via the photodetection element; and
drive the optical path length difference adjustment unit on the basis of an analysis result of the OCT signal and a change in the focus state of the observation optical system.

8. The observation system according to any one of claims 1 to 7, wherein
the processor is adapted to:
analyze an OCT signal acquired via the photodetection element; and
drive the OCT focus adjustment unit on the basis of an analysis result of the OCT signal and a change in the focus state of the observation optical system.

9. The observation system according to any one of claims 1 to 8, wherein
the processor is adapted to:
acquire an optical parameter of the observation optical system; and
drive at least one of the optical path length difference adjustment unit and the OCT focus adjustment unit on the basis of the acquired optical parameter.

10. The observation system according to any one of claims 1 to 9, wherein
the processor is adapted to change a drive amount of at least one of the optical path length difference adjustment unit and the OCT focus adjustment unit, depending on whether a wide angle observation unit (37) is being used, the wide angle observation unit being adapted to widen an observation angle of view of the ocular fundus of a patient's eye that is the biological object.

11. The observation system according to any one of claims 1 to 10, wherein
the processor is adapted to:
acquire a deviation amount, in a depth direction along a luminous flux, between an observation target position and an OCT target position, the observation target position being a reference for focusing the focus of the observation optical system, and the OCT target position being a reference for acquiring the OCT signal; and
drive at least one of the optical path length difference adjustment unit and the OCT focus adjustment unit in accordance with the acquired deviation amount.

12. An observation control program executed by a control unit (60) adapted to control an observation system (100, 101), the observation system comprising a surgical microscope (1) and an OCT unit (40), and the OCT unit being adapted to acquire an OCT signal,
wherein
the surgical microscope includes:
an observation optical system (30) adapted to optically guide an observation luminous flux from a biological object being an observation object; and
an observation focus adjustment unit (32) provided on an optical path of the observation luminous flux in the observation optical system, the observation focus adjustment unit being adapted to adjust a focus of the observation optical system,
the OCT unit includes:
an OCT light source (41);
a light splitter (42) adapted to split a luminous flux emitted from the OCT light source into a measurement luminous flux and a reference luminous flux;
a photodetection element (59) adapted to receive an interference light obtained by synthesis of the reference luminous flux and a measurement luminous flux reflected by the biological object; and
an optical path length difference adjustment unit (56) provided on at least one of an optical path of the measurement luminous flux and an optical path of the reference luminous flux, the optical path length difference adjustment unit being adapted to adjust an optical path length difference between the measurement luminous flux and the reference luminous flux,
the observation control program, when executed by a processor of the control unit, causes the control unit to acquire a focus state of the observation optical system and to perform an optical path length difference adjustment step of driving the optical path length difference adjustment unit of the OCT unit in accordance with a change in the focus state of the observation optical system,
the OCT unit further includes:
an OCT focus adjustment unit (49) provided on the optical path of the measurement luminous flux, the OCT focus adjustment unit being adapted to adjust a focus of an optical system that optically guides the measurement luminous flux, and
the observation control program, when executed by the processor of the control unit, causes the control unit to perform an OCT focus adjustment step of driving the OCT focus adjustment unit in accordance with a change in the focus state of the observation optical system.

## Patentansprüche

1. Beobachtungssystem (100, 101), aufweisend:
ein Operationsmikroskop (1),
eine OCT-Einheit (40), die angepasst ist, um ein OCT-Signal zu erlangen, und
einen Prozessor (61),
wobei
das Operationsmikroskop aufweist:
ein optisches Beobachtungssystem (30), das angepasst ist, um einen Beobachtungslichtstrom von einem biologischen Objekt, das ein Beobachtungsobjekt ist, optisch zu leiten, und
eine Beobachtungsfokus-Einstelleinheit (32), die auf einem optischen Pfad des Beobachtungslichtstroms in dem optischen Beobachtungssystem vorgesehen ist, wobei die Beobachtungsfokus-Einstelleinheit angepasst ist, um einen Fokus des optischen Beobachtungssystems einzustellen,
die OCT-Einheit aufweist:
eine OCT-Lichtquelle (41),
einen Lichtteiler (42), der angepasst ist, um einen von der OCT-Lichtquelle emittierten Lichtstrom in einen Messlichtstrom und einen Referenzlichtstrom aufzuteilen,
ein Fotodetektionselement (59), das angepasst ist, um ein Interferenzlicht zu empfangen, das durch Synthese des Referenzlichtstroms und eines Messlichtstroms, der von dem biologischen Objekt reflektiert wird, erhalten wird, und
eine Optischer-Pfad-Längendifferenz-Einstelleinheit (56), die auf mindestens einem von einem optischen Pfad des Messlichtstroms und einem optischen Pfad des Referenzlichtstroms vorgesehen ist, wobei die Optischer-Pfad-Längendifferenz-Einstelleinheit angepasst ist, um eine Optischer-Pfad-Längendifferenz zwischen dem Messlichtstrom und dem Referenzlichtstrom einzustellen,
der Prozessor angepasst ist, um einen Fokuszustand des optischen Beobachtungssystems zu erlangen und die Optischer-Pfad-Längendifferenz-Einstelleinheit der OCT-Einheit gemäß einer Änderung eines Fokuszustands des optischen Beobachtungssystems anzusteuern,
die OCT-Einheit ferner aufweist:
eine OCT-Fokus-Einstelleinheit (49), die auf dem optischen Pfad des Messlichtstroms vorgesehen ist, wobei die OCT-Fokus-Einstelleinheit angepasst ist, um einen Fokus eines optischen Systems einzustellen, das den Messlichtstrom optisch leitet,
**dadurch gekennzeichnet, dass**
der Prozessor angepasst ist, um die OCT-Fokus-Einstelleinheit gemäß einer Änderung des Fokuszustandes des optischen Beobachtungssystems anzusteuern.

2. Beobachtungssystem gemäß Anspruch 1, wobei der Prozessor angepasst ist, um:
den Fokuszustand des optischen Beobachtungssystems des Operationsmikroskops zu erlangen und
die Beobachtungsfokus-Einstelleinheit und die Optischer-Pfad-Längendifferenz-Einstelleinheit auf der Grundlage eines Erlangungsergebnisses des Fokuszustands des optischen Beobachtungssystems anzusteuern.

3. Beobachtungssystem gemäß Anspruch 2, wobei
das Operationsmikroskop ferner eine Abbildungsvorrichtung (36R, 36L) aufweist, die angepasst ist, um ein mikroskopisches Bild des biologischen Objekts durch Empfangen des Beobachtungslichtstroms, der durch das optische Beobachtungssystem optisch geleitet wird, zu erfassen, und
der Prozessor angepasst ist, um:
eine Anzeige (67) zu veranlassen, das von der Abbildungsvorrichtung erfasste mikroskopische Bild anzuzeigen und
den Fokuszustand des optischen Beobachtungssystems auf der Grundlage eines Signals von der Abbildungsvorrichtung zu erlangen.

4. Beobachtungssystem gemäß Anspruch 3, wobei
der Prozessor angepasst ist, um:
einen Interessensbereich (81) innerhalb eines Bereichs des mikroskopischen Bildes festzulegen, das von der Abbildungsvorrichtung erfasst wird, und
den Fokuszustand des optischen Beobachtungssystems auf der Grundlage eines Signals für einen Evaluierungsbereich (82) zu erlangen, wobei der Evaluierungsbereich ein Bereich des mikroskopischen Bildes ist, der den festgelegten Interessenbereich aufweist.

5. Beobachtungssystem gemäß Anspruch 4, wobei
der Prozessor angepasst ist, um eine Position (83) festzulegen, an der die OCT-Einheit bewirkt, dass der Messlichtstrom auf der Grundlage von mindestens einem von dem Interessenbereich und dem Evaluierungsbereich abtastet.

6. Beobachtungssystem gemäß einem der Ansprüche 4 und 5, wobei
der Prozessor angepasst ist, um:
eine Bewegung eines in dem mikroskopischen Bild erfassten Subjekts durch Durchführen einer Bildverarbeitung an dem von der Abbildungsvorrichtung erfassten mikroskopischen Bild zu detektieren, und
zu veranlassen, dass eine Position des Evaluierungsbereichs in dem mikroskopischen Bild gemäß der detektierten Bewegung des Subjekts verfolgt wird.

7. Beobachtungssystem gemäß irgendeinem der Ansprüche 1 bis 6, wobei
der Prozessor angepasst ist, um:
ein über das Fotodetektionselement erlangtes OCT-Signal zu analysieren und
die Optischer-Pfad-Längendifferenz-Einstelleinheit auf der Grundlage eines Analyseergebnisses des OCT-Signals und einer Änderung des Fokuszustands des optischen Beobachtungssystems anzusteuern.

8. Beobachtungssystem gemäß irgendeinem der Ansprüche 1 bis 7, wobei
der Prozessor angepasst ist, um:
ein über das Fotodetektionselement erlangtes OCT-Signal zu analysieren und
die OCT-Fokus-Einstelleinheit auf der Grundlage eines Analyseergebnisses des OCT-Signals und einer Änderung des Fokuszustands des optischen Beobachtungssystems anzusteuern.

9. Beobachtungssystem gemäß irgendeinem der Ansprüche 1 bis 8, wobei
der Prozessor angepasst ist, um:
einen optischen Parameter des optischen Beobachtungssystems zu erlangen und
mindestens eine der Optischer-Pfad-Längendifferenz-Einstelleinheit und der OCT-Fokus-Einstelleinheit auf der Grundlage des erlangten optischen Parameters anzusteuern.

10. Beobachtungssystem gemäß einem der Ansprüche 1 bis 9, wobei
der Prozessor angepasst ist, um einen Ansteuerungsbetrag von mindestens einer von der Optischer-Pfad-Längendifferenz-Einstelleinheit und der OCT-Fokus-Einstelleinheit in Abhängigkeit davon zu ändern, ob eine Weitwinkel-Beobachtungseinheit (37) verwendet wird, wobei die Weitwinkel-Beobachtungseinheit angepasst ist, um einen Beobachtungswinkel des Augenhintergrunds eines Patientenauges, der das biologische Objekt ist, zu erweitern.

11. Beobachtungssystem gemäß irgendeinem der Ansprüche 1 bis 10, wobei
der Prozessor angepasst ist, um:
einen Abweichungsbetrag in einer Tiefenrichtung entlang eines Lichtstroms zwischen einer Beobachtungszielposition und einer OCT-Zielposition zu erlangen, wobei die Beobachtungszielposition eine Referenz zum Fokussieren des Fokus des optischen Beobachtungssystems ist und die OCT-Zielposition eine Referenz zum Erlangen des OCT-Signals ist, und
mindestens eine von der Optischer-Pfad-Längendifferenz-Einstelleinheit und der OCT-Fokus-Einstelleinheit gemäß dem erlangten Abweichungsbetrag anzusteuern.

12. Beobachtungssteuerprogramm, das von einer Steuereinheit (60) ausgeführt wird, die zum Steuern eines Beobachtungssystems (100, 101) angepasst ist, wobei das Beobachtungssystem ein Operationsmikroskop (1) und eine OCT-Einheit (40) aufweist und die OCT-Einheit angepasst ist, um ein OCT-Signal zu erlangen,
wobei
das Operationsmikroskop aufweist:
ein optisches Beobachtungssystem (30), das angepasst ist, um einen Beobachtungslichtstrom von einem biologischen Objekt, das ein Beobachtungsobjekt ist, optisch zu leiten, und
eine Beobachtungsfokus-Einstelleinheit (32), die auf einem optischen Pfad des Beobachtungslichtstroms in dem optischen Beobachtungssystem vorgesehen ist, wobei die Beobachtungsfokus-Einstelleinheit angepasst ist, um einen Fokus des optischen Beobachtungssystems einzustellen,
die OCT-Einheit aufweist:
eine OCT-Lichtquelle (41),
einen Lichtteiler (42), der angepasst ist, um einen von der OCT-Lichtquelle emittierten Lichtstrom in einen Messlichtstrom und einen Referenzlichtstrom aufzuteilen,
ein Fotodetektionselement (59), das angepasst ist, um ein Interferenzlicht zu empfangen, das durch Synthese des Referenzlichtstroms und eines Messlichtstroms, der von dem biologischen Objekt reflektiert wird, erhalten wird, und
eine Optischer-Pfad-Längendifferenz-Einstelleinheit (56), die auf mindestens einem von einem optischen Pfad des Messlichtstroms und einem optischen Pfad des Referenzlichtstroms vorgesehen ist, wobei die Optischer-Pfad-Längendifferenz-Einstelleinheit angepasst ist, um eine Optischer-Pfad-Längendifferenz zwischen dem Messlichtstrom und dem Referenzlichtstrom einzustellen,
das Beobachtungssteuerprogramm, wenn es von einem Prozessor der Steuereinheit ausgeführt wird, die Steuereinheit veranlasst, einen Fokuszustand des optischen Beobachtungssystems zu erlangen und einen Optischer-Pfad-Längendifferenz-Einstellschritt zum Ansteuern der Optischer-Pfad-Längendifferenz-Einstelleinheit der OCT-Einheit gemäß einer Änderung des Fokuszustandes des optischen Beobachtungssystems durchzuführen,
die OCT-Einheit ferner aufweist:
eine OCT-Fokus-Einstelleinheit (49), die auf dem optischen Pfad des Messlichtstroms vorgesehen ist, wobei die OCT-Fokus-Einstelleinheit angepasst ist, um einen Fokus eines optischen Systems einzustellen, das den Messlichtstrom optisch leitet, und
das Beobachtungssteuerprogramm, wenn es durch den Prozessor der Steuereinheit ausgeführt wird, die Steuereinheit veranlasst, einen OCT-Fokus-Einstellschritt des Ansteuerns der OCT-Fokus-Einstelleinheit gemäß einer Änderung des Fokuszustands des optischen Beobachtungssystems durchzuführen.

## Revendications

1. Système d'observation (100, 101) comprenant :
un microscope chirurgical (1) ;
une unité OCT (40) adaptée pour acquérir un signal OCT ; et
un processeur (61),
dans lequel
le microscope chirurgical comprend :
un système optique d'observation (30) adapté pour guider optiquement un flux lumineux d'observation provenant d'un objet biologique qui est un objet d'observation ; et
une unité d'ajustement de foyer d'observation (32) prévue sur un chemin optique du flux lumineux d'observation dans le système optique d'observation, l'unité d'ajustement de foyer d'observation étant adaptée pour ajuster un foyer du système optique d'observation,
l'unité OCT comprend :
une source de lumière OCT (41) ;
un diviseur de lumière (42) adapté pour diviser un flux lumineux émis par la source de lumière OCT en un flux lumineux de mesure et un flux lumineux de référence ;
un élément de photodétection (59) adapté pour recevoir une lumière d'interférence obtenue par synthèse du flux lumineux de référence et d'un flux lumineux de mesure réfléchi par l'objet biologique ; et
une unité d'ajustement de différence de longueur de chemin optique (56) prévue sur au moins l'un parmi un chemin optique du flux lumineux de mesure et un chemin optique du flux lumineux de référence, l'unité d'ajustement de différence de longueur de chemin optique étant adaptée pour ajuster une différence de longueur de chemin optique entre le flux lumineux de mesure et le flux lumineux de référence,
le processeur est adapté pour acquérir un état de foyer du système optique d'observation et pour entraîner l'unité d'ajustement de différence de longueur de chemin optique de l'unité OCT en fonction d'un changement d'état de foyer du système optique d'observation,
l'unité OCT comprend en outre :
une unité d'ajustement de foyer OCT (49) prévue sur le chemin optique du flux lumineux de mesure, l'unité d'ajustement de foyer OCT étant adaptée pour ajuster un foyer d'un système optique qui guide optiquement le flux lumineux de mesure,
**caractérisé en ce que**
le processeur est adapté pour entraîner l'unité d'ajustement de foyer OCT en fonction d'un changement de l'état de foyer du système optique d'observation.

2. Système d'observation selon la revendication 1, dans lequel
le processeur est adapté pour :
acquérir l'état de foyer du système optique d'observation du microscope chirurgical ; et
entraîner l'unité d'ajustement de foyer d'observation et l'unité d'ajustement de différence de longueur de chemin optique sur la base d'un résultat d'acquisition de l'état de foyer du système optique d'observation.

3. Système d'observation selon la revendication 2, dans lequel
le microscope chirurgical comprend en outre un dispositif d'imagerie (36R, 36L) adapté pour capturer une image microscopique de l'objet biologique en recevant le flux lumineux d'observation guidé optiquement par le système optique d'observation, et
le processeur est adapté pour :
amener un écran (67) à afficher l'image microscopique capturée par le dispositif d'imagerie ; et
acquérir l'état de foyer du système optique d'observation sur la base d'un signal provenant du dispositif d'imagerie.

4. Système d'observation selon la revendication 3, dans lequel
le processeur est adapté pour :
définir une région d'intérêt (81) dans une région de l'image microscopique capturée par le dispositif d'imagerie ; et
acquérir l'état de foyer du système optique d'observation sur la base d'un signal pour une région d'évaluation (82), la région d'évaluation étant une région, de l'image microscopique, qui comprend la région d'intérêt définie.

5. Système d'observation selon la revendication 4, dans lequel
le processeur est adapté pour définir une position (83) à laquelle l'unité OCT fait balayer le flux lumineux de mesure, sur la base d'au moins l'une de la région d'intérêt et de la région d'évaluation.

6. Système d'observation selon l'une ou l'autre des revendications 4 et 5, dans lequel
le processeur est adapté pour :
détecter un mouvement d'un sujet capturé dans l'image microscopique en effectuant un traitement d'image sur l'image microscopique capturée par le dispositif d'imagerie ; et
faire en sorte qu'une position de la région d'évaluation dans l'image microscopique soit suivie en fonction du mouvement détecté du sujet.

7. Système d'observation selon l'une quelconque des revendications 1 à 6, dans lequel
le processeur est adapté pour :
analyser un signal OCT acquis via l'élément de photodétection ; et
entraîner l'unité d'ajustement de différence de longueur de chemin optique sur la base d'un résultat d'analyse du signal OCT et d'un changement de l'état de foyer du système optique d'observation.

8. Système d'observation selon l'une quelconque des revendications 1 à 7, dans lequel
le processeur est adapté pour :
analyser un signal OCT acquis via l'élément de photodétection ; et
entraîner l'unité d'ajustement de foyer OCT sur la base d'un résultat d'analyse du signal OCT et d'un changement de l'état de foyer du système optique d'observation.

9. Système d'observation selon l'une quelconque des revendications 1 à 8, dans lequel
le processeur est adapté pour :
acquérir un paramètre optique du système optique d'observation ; et
entraîner au moins l'une de l'unité d'ajustement de différence de longueur de chemin optique et de l'unité d'ajustement de foyer OCT sur la base du paramètre optique acquis.

10. Système d'observation selon l'une quelconque des revendications 1 à 9, dans lequel
le processeur est adapté pour modifier une quantité d'entraînement d'au moins l'une de l'unité d'ajustement de différence de longueur de chemin optique et de l'unité d'ajustement de foyer OCT, selon qu'une unité d'observation grand angle (37) est utilisée, l'unité d'observation grand angle étant adaptée pour élargir un angle de vue d'observation du fond de l'œil d'un patient qui est l'objet biologique.

11. Système d'observation selon l'une quelconque des revendications 1 à 10, dans lequel
le processeur est adapté pour :
acquérir une quantité d'écart, dans une direction de profondeur le long d'un flux lumineux, entre une position cible d'observation et une position cible OCT, la position cible d'observation étant une référence pour focaliser le foyer du système optique d'observation, et la position cible OCT étant une référence pour acquérir le signal OCT ; et
entraîner au moins l'une de l'unité d'ajustement de différence de longueur de chemin optique et de l'unité d'ajustement de foyer OCT en fonction de la quantité d'écart acquise.

12. Programme de commande d'observation exécuté par une unité de commande (60) adaptée pour commander un système d'observation (100, 101), le système d'observation comprenant un microscope chirurgical (1) et une unité OCT (40), et l'unité OCT étant adaptée pour acquérir un signal OCT,
dans lequel
le microscope chirurgical comprend :
un système optique d'observation (30) adapté pour guider optiquement un flux lumineux d'observation provenant d'un objet biologique qui est un objet d'observation ; et
une unité d'ajustement de foyer d'observation (32) prévue sur un chemin optique du flux lumineux d'observation dans le système optique d'observation, l'unité d'ajustement de foyer d'observation étant adaptée pour ajuster un foyer du système optique d'observation,
l'unité OCT comprend :
une source de lumière OCT (41) ;
un diviseur de lumière (42) adapté pour diviser un flux lumineux émis par la source de lumière OCT en un flux lumineux de mesure et un flux lumineux de référence ;
un élément de photodétection (59) adapté pour recevoir une lumière d'interférence obtenue par synthèse du flux lumineux de référence et d'un flux lumineux de mesure réfléchi par l'objet biologique ; et
une unité d'ajustement de différence de longueur de chemin optique (56) prévue sur au moins l'un d'un chemin optique du flux lumineux de mesure et d'un chemin optique du flux lumineux de référence, l'unité d'ajustement de différence de longueur de chemin optique étant adaptée pour ajuster une différence de longueur de chemin optique entre le flux lumineux de mesure et le flux lumineux de référence,
le programme de commande d'observation, lorsqu'il est exécuté par un processeur de l'unité de commande, amène l'unité de commande à acquérir un état de foyer du système optique d'observation et à exécuter une étape d'ajustement de différence de longueur de chemin optique consistant à entraîner l'unité d'ajustement de différence de longueur de chemin optique de l'unité OCT en fonction d'un changement de l'état de foyer du système optique d'observation,
l'unité OCT comprend en outre :
une unité d'ajustement de foyer OCT (49) prévue sur le chemin optique du flux lumineux de mesure, l'unité d'ajustement de foyer OCT étant adaptée pour ajuster un foyer d'un système optique qui guide optiquement le flux lumineux de mesure, et
le programme de commande d'observation, lorsqu'il est exécuté par le processeur de l'unité de commande, amène l'unité de commande à exécuter une étape d'ajustement de foyer OCT consistant à entraîner l'unité d'ajustement de foyer OCT en fonction d'un changement de l'état de foyer du système optique d'observation.
